# EUROPÄISCHE PATENTSCHRIFT

(11) **EP 4 108 158 B1**
(45) Veröffentlichungstag und Bekanntmachung des Hinweises auf die Patenterteilung: **25.02.2026**
(21) Anmeldenummer: 22177816.0
(22) Anmeldetag: 08.06.2022
(51) Int. Cl.: A61B 1/05, A61B 1/00, A61B 1/267

(54) **AUFSATZBAUGRUPPE SOWIE VIDEOLARYNGOSKOPSYSTEM**
ATTACHMENT ASSEMBLY AND VIDEO LARYNGOSCOPE SYSTEM
MODULE RAPPORTÉ, AINSI QUE SYSTÈME DE VIDÉO-LARYNGOSCOPE

(30) Priorität: 23.06.2021 DE 102021116266
(43) Veröffentlichungstag der Anmeldung: 28.12.2022
(73) Patentinhaber: HEINE Optotechnik GmbH & Co. KG, 82205 Gilching (DE)
(72) Erfinder: MARTIN, Timo, 86911 Dettenschwang (DE); MICHEL, Felix, 81241 München (DE); ENTSFELLNER, Konrad, 82205 Gilching (DE)
(74) Vertreter: Flach Bauer & Partner Patentanwälte mbB

(56) Entgegenhaltungen:
- WO-A1-2022/081509
- WO-A2-2016/074894
- US-A1- 2014 160 261

## Beschreibung

Die Erfindung betrifft eine Aufsatzbaugruppe für einen Kameraarm eines Videolaryngoskops sowie ein Videolaryngoskopsystem mit einem Videolaryngoskop mit einem Kameraarm.

Videolaryngoskope mit Kameraarm sind bekannt. Bei solchen Videolaryngoskopen werden Spatel für Laryngoskopien auf den Kameraarm aufgesetzt, um die Kamera des Kameraarms und auch den Kameraarm selbst vor Verunreinigungen bei der Laryngoskopie zu schützen. Dadurch wird der Reinigungsaufwand des Kameraarms und somit des gesamten Videolaryngoskops verringert. Die Spatel werden nach jeder Patientenuntersuchung ausgetauscht und, im Falle von Mehrwegspateln, sterilisiert oder, im Falle von Einwegspateln, entsorgt.

Es sind hierzu Einwegspatel bekannt, die einstückig ausgeführt sind und einen Kanal aufweisen, in den der Kameraarm eingeführt wird. Der Kanal ist dabei patientenseitig geschlossen, sodass die Kamera und der Kameraarm patientenseitig vollständig durch den Spatel eingekapselt sind. Das Blickfeld der Kamera muss somit am patientenseitigen Ende des Kanals durch den Spatel verlaufen, wodurch strenge Bedingungen an die Materialeigenschaften des Spatels gestellt werden. Insbesondere musst ein Spatel aus transparentem Material hergestellt sein. Dies führt zu einer verringerten Flexibilität in der Herstellung von Einwegspatel, wobei insbesondere keine Einwegspatel aus recyceltem Material hergestellt werden können, da recyceltes Material nicht die notwendigen optischen Eigenschaften aufweist.

Beispielsweise offenbart die WO 2016/074894 A1 ein Videolaryngoskop mit einem Spatel, dessen Krümmung einstellbar ist. Der Spatel hat einen Hohlraum, durch den sich ein Führungsschlauch erstreckt und der den Hohlraum nach vorne hin abschließt. Im Führungsschlauch wiederum verläuft ein Kameraarm, der durch den Führungsschlauch vor Körperflüssigkeiten geschützt ist.

Es ist daher Aufgabe der Erfindung, ein Videolaryngoskopsystem sowie eine Aufsatzbaugruppe für ein Videolaryngoskop bereitzustellen, bei denen das Material des Spatels geringeren Anforderungen, insbesondere optischen Anforderungen genügen muss.

Die Aufgabe wird gelöst durch eine Aufsatzbaugruppe für einen Kameraarm eines Videolaryngoskops mit einem Spatel und einer Schutzkappe. Der Spatel weist einen Grundkörper mit einem Griffende und einem zu dem Griffende entgegengesetzten Patientenende sowie einen im Grundkörper ausgebildeten Kanal zur Aufnahme des Kameraarms auf, wobei der Kanal sowohl zum Griffende hin als auch zum Patientenende hin geöffnet ist. Die Schutzkappe ist ein vom Spatel separates Bauteil, ist in den Kanal einführbar und weist einen zumindest teilweise transparenten Schutzabschnitt auf, wobei die Schutzkappe dazu ausgebildet ist, dass eine Spitze des Kameraarms des Videolaryngoskops in den Schutzabschnitt eingreifen kann und vom Schutzabschnitt abgedeckt wird. Die Schutzkappe ist im Ausgangszustand der Aufsatzbaugruppe innerhalb des Kanals angeordnet und weist einen Befestigungsabschnitt auf, der die Schutzkappe gegenüber dem Grundkörper in einer Ausgangsposition positioniert.

Als separates Bauteil kann die Schutzkappe insbesondere relativ zum Grundkörper bewegt werden.

Insbesondere verläuft das Blickfeld der Kamera an der Spitze des Kameraarms durch den Schutzabschnitt und nicht durch den Grundkörper, wenn die Spitze des Kameraarms in den Schutzabschnitt eingreift.

Durch die vom Spatel separate Schutzkappe wird erreicht, dass der Spatel den Kameraarm und insbesondere die Kamera an der Spitze des Kameraarms nicht mehr verdecken muss, sodass der Spatel selbst geringeren Materialanforderungen genügen kann, insbesondere muss der Spatel nicht mehr transparent sein.

Außerdem wurde erkannt, dass der Kanal am patientenseitigen Ende nicht vollständig dicht sein muss, um den Kameraarm hinreichend vor Verunreinigungen zu schützen.

Der Grundkörper kann eine Macintosh-, Miller-, Dörges- oder McCoy-Form haben. Beispielsweise besteht der Spatel vollständig aus dem Grundkörper.

Der Schutzabschnitt kann auch vollständig transparent sein.

Insbesondere ist die Schutzkappe kein Teil des Kameraarms, sondern ein Teil der vom Kameraarm separaten Aufsatzbaugruppe. Die Schutzkappe kommt beispielsweise erst beim oder kurz vor dem Einschieben des Kameraarm in den Kanal mit dem Kameraarm in Kontakt.

In einer Ausführungsform ist die Schutzkappe im Benutzungszustand der Aufsatzbaugruppe im Bereich des patientenseitigen Endes des Kanals angeordnet, insbesondere erstreckt sich die Schutzkappe zumindest teilweise durch die patientenseitige Öffnung des Kanals. Auf diese Weise ist sichergestellt, dass die Schutzkappe den Kameraarm schützt und das Blickfeld der Kamera wie vorgesehen verläuft.

Beispielsweise verklemmt der Befestigungsabschnitt die Schutzkappe gegenüber dem Grundkörper in einer Ausgangsposition im Kanal.

Die Positionierung ist beispielsweise werkseitig vorgenommen und insbesondere lösbar, zum Beispiel durch den Kameraarm.

Durch die werkseitige Positionierung der Schutzkappe im Ausgangszustand wird die Benutzung durch den Benutzer vereinfacht. Zugleich wird gewährleistet, dass die Schutzkappe korrekt auf dem Kameraarm sitzt, da der Benutzer den Kameraarm weiter als die Ausgangsposition in den Kanal einführen wird.

Die Ausgangsposition kann zwischen der griffseitigen Öffnung und der patientenseitigen Öffnung des Kanals liegen und/oder sich von der Position der Schutzkappe im Benutzungszustand unterscheiden.

In einer Ausgestaltung der Erfindung weist der Befestigungsabschnitt wenigstens zwei, insbesondere vier Flügel auf, die sich von entgegengesetzten Seiten des Schutzabschnitts aus erstrecken, wobei die wenigstens zwei Flügel an entsprechenden Kanalwänden des Kanals anliegen und/oder wobei sich die Flügel weiter nach außen erstrecken als der Schutzabschnitt. Durch die Flügel wird die Schutzkappe auf einfache Weise im Kanal positioniert.

Die Flügel sind insbesondere elastisch, sodass sie in der Ausgangsposition gegen die Kanalwände vorgespannt sein können.

Außen kann in Bezug auf eine Mittellinie der Aufnahme oder des Kanals als radial nach außen verstanden werden.

Zur genauen Festlegung der Position der Schutzkappe und damit des Blickfelds der Kamera, kann im Kanal an seinem patientenseitigen Ende wenigstens ein Anschlag vorgesehen sein, an dem die Schutzkappe, insbesondere der Schutzabschnitt, im Benutzungszustand anliegt.

In einer Ausführungsform weist der Schutzabschnitt eine transparente Frontscheibe und wenigstens zwei, insbesondere vier Wände auf, wobei sich die Wände von der Frontscheibe aus erstrecken und eine Aufnahme für die Spitze des Kameraarms bilden, insbesondere wobei die Wände parallel verlaufen und/oder die Frontscheibe schräg zu den Wänden angeordnet ist. Durch die Aufnahme wird die Spitze des Kameraarm zuverlässig positioniert.

Die Frontscheibe kann die Aufnahme patientenseitig vollständig verschließen.

Beispielsweise erstrecken sich die Flügel von den Wänden aus, insbesondere an der von der Frontscheibe abgewandten Seite der Wände.

Um den Spatel einfach und/oder umweltschonend herzustellen, kann der Spatel aus einem intransparenten Material und/oder aus einem zumindest überwiegend, insbesondere vollständig recycleten Material sein.

In einer Ausgestaltung ist die patientenseitige Öffnung des Kanals zwischen dem Griffende und dem Patientenende des Grundkörpers angeordnet, insbesondere im mittleren Drittel zwischen dem Griffende und dem Patientenende, wodurch der Spatel am Patientenende dünn ausgeführt ist, um das Verletzungsrisiko zu verringern.

Der Grundkörper hat beispielsweise einen Vorsprung zwischen Patientenende und Ende des Kanals.

Zum zuverlässigen Schutz des Kameraarms kann der Kanal entlang seines Umfangs geschlossen sein, insbesondere über die gesamte Länge des Kanals.

Der Querschnitt des Kanals kann sich von der griffseitigen Öffnung des Kanals zur patientenseitigen Öffnung des Kanals hin verkleinern, insbesondere die Höhe des Querschnitts kann sich verkleinert, wodurch das Einführen des Kameraarms in den Kanal vereinfacht wird.

In einer Ausgestaltung hat der Grundkörper in Querrichtung zur Einführrichtung eine Breite, wobei sich der Kanal nur über einen Teil der Breite, insbesondere gleich oder weniger als die Hälfte der Breite erstreckt. Auf diese Weise wird eine klassische visuelle Untersuchung, d.h. ohne Kameraunterstützung, ermöglicht.

Zur kostengünstigen Herstellung kann der Grundkörper und/oder die Schutzkappe einstückig ausgeführt sein.

Zum Beispiel sind der Grundkörper und/oder die Schutzkappe je ein Spritzgussteil.

Ferner wird die Aufgabe gelöst durch ein Videolaryngoskopsystem mit einem Videolaryngoskop, das einen Kameraarm aufweist, und einer zuvor beschriebenen Aufsatzbaugruppe für den Kameraarm.

Die für die Aufsatzbaugruppe beschriebenen Merkmale und Vorteile gelten gleichermaßen für das Videolaryngoskopsystem und umgekehrt.

Beispielsweise weist der Kameraarm eine Spitze mit einer Kamera auf, wobei im Benutzungszustand die Spitze in die Schutzkappe, insbesondere die Aufnahme der Schutzkappe, eingreift und der Kameraarm sich durch den Kanal erstreckt. Auf diese Weise werden sowohl die Kamera als auch der Kameraarm hinreichend vor Verunreinigung geschützt.

Insbesondere verläuft das Blickfeld der Kamera durch die Frontscheibe in Richtung zum patientenseitigen Ende des Grundkörpers hin.

Der Kameraarm hält beispielsweise sowohl den Spatel also auch die Schutzkappe innerhalb des Spatels.

In einer Ausführungsform ist das Videolaryngoskopsystem derart ausgebildet, dass der Kameraarm die Schutzkappe beim Einführen des Kameraarms in den Kanal des Spatels aus der Ausgangsposition mitnimmt und zur patientenseitigen Öffnung des Kanals schiebt, bis der Benutzungszustand erreicht ist, wodurch die Verwendung des Spatels äußerst einfach ist.

In einer Ausgestaltung weist das Videolaryngoskopsystem einen vom Kameraarm und dem Spatel separaten Spender für die Schutzkappe auf, wobei der Spender ein Gehäuse mit einer Spendeöffnung aufweist, und wobei innerhalb des Gehäuses im Bereich der Spendeöffnung die Schutzkappe vorgesehen ist. Auf diese Weise können die Schutzkappen separat vom Spatel bereitgestellt werden.

Weitere Vorteile und Merkmale der Erfindung ergeben sich aus der nachfolgenden Beschreibung sowie aus den beigefügten Zeichnungen, auf die Bezug genommen wird. In den Zeichnungen zeigen:
- Fig. 1:: ein erfindungsgemäßes Videolaryngoskopsystem mit einer erfindungsgemäßen Aufsatzbaugruppe in perspektivische Ansicht,
- Figs. 2, 3, 4:: einen Spatel der erfindungsgemäßen Aufsatzbaugruppe gemäß Figur 1 in einer perspektivischen Ansicht, einer Vorderansicht bzw. einer Schnittansicht,
- Figs. 5, 6, 7:: eine Schutzkappe der erfindungsgemäßen Aufsatzbaugruppe gemäß Figur 1 in einer perspektivischen Ansicht, einer Vorderansicht bzw. in einer Schnittansicht,
- Fig. 8:: die erfindungsgemäße Aufsatzbaugruppe gemäß Figur 1 im Schnitt im Ausgangszustand, und
- Figs. 9, 10:: die Aufsatzbaugruppe gemäß Figur 8, in die der Kameraarm des Videolaryngoskops eingeführt wird, im Ausgangszustand bzw. im Benutzungszustand.

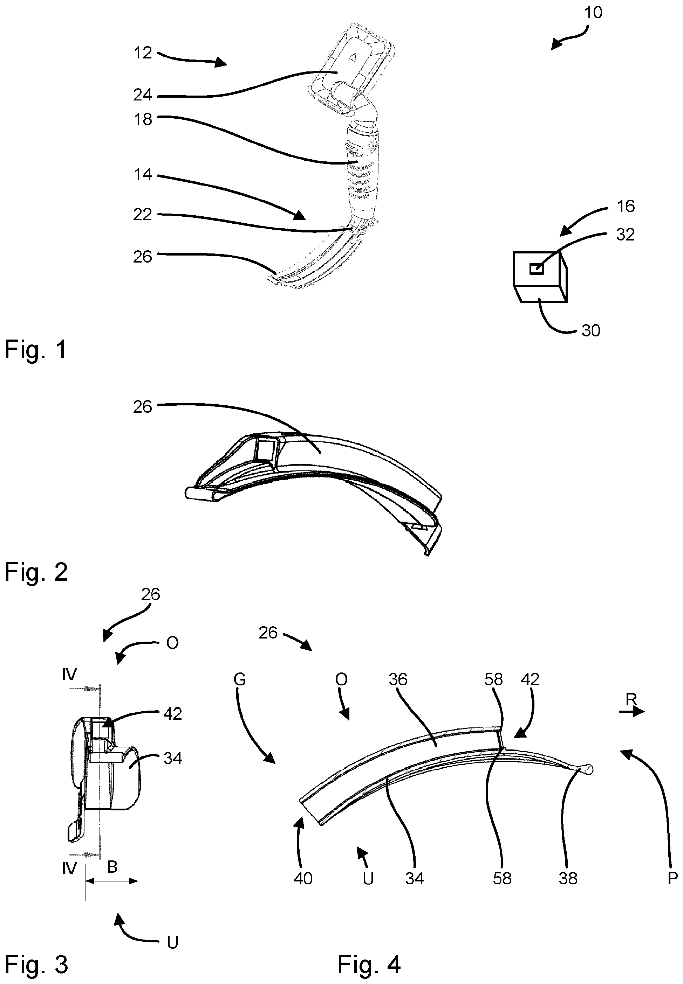

Figur 1 zeigt ein Videolaryngoskopsystem 10 mit einem Videolaryngoskop 12, einer Aufsatzbaugruppe 14 sowie einem optionalen Spender 16.

Das Videolaryngoskop 12 hat einen Griff 18 und einen sich davon erstreckenden Kameraarm 20, an dessen vom Griff 18 abgewandten Spitze eine Kamera 22 angeordnet ist.

Außerdem kann am Griff 18 ein Bildschirm 24 vorgesehen sein, der das mit der Kamera 22 aufgenommene Bild wiedergibt.

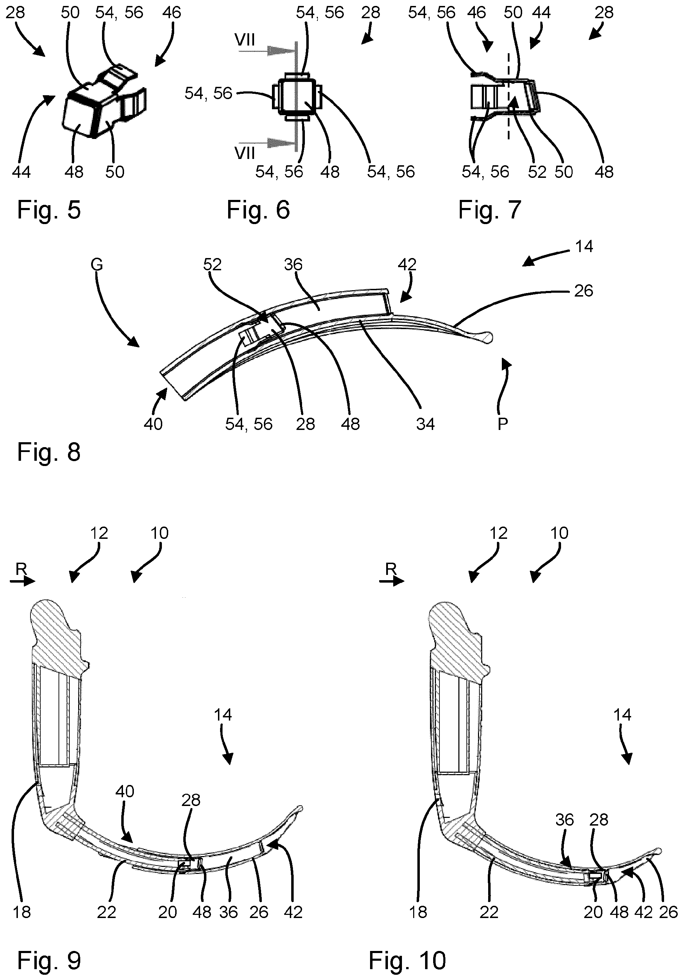

Die Aufsatzbaugruppe 14 weist einen Spatel 26 sowie eine Schutzkappe 28 (Fig. 5) für die Kamera 22 auf, wobei die Schutzkappe 28 am Kameraarm 20 befestigt ist.

Der optionale Spender 16 ist ein Spender für Schutzkappen 28 und hat ein Gehäuse 30, in dem eine Spendeöffnung 32 ausgebildet ist.

In den Figuren 2, 3, 4 ist der Spatel 26 der Aufsatzbaugruppe 14 in einer perspektivischen Ansicht, in einer Vorderansicht auf das patientenseitige Ende P des Spatel 26 bzw. in einer Schnittansicht dargestellt.

Der Spatel 26 weist einen Grundkörper 34 auf, insbesondere besteht er aus dem Grundkörper 34. Beispielsweise ist der Spatel 26 ein Einwegwegprodukt, also ein Einmalspatel.

Der Grundkörper 34 ist einstückig ausgeführt, beispielsweise als Spritzgussteil. Das Material des Grundkörpers 34 ist intransparent und beispielsweise überwiegend, insbesondere vollständig recycelt.

Der Grundkörper 34 hat ein Griffende G, das dem Griff 18 des Videolaryngoskops 12 zugewandt ist, sowie ein Patientenende, das dem Griffende G entgegengesetzt ist, d.h. von Griff 18 abgewandt ist, und dem Patienten zugewandt ist bzw. in den Patienten eingeführt wird.

Zudem hat der Grundkörper 34 eine Unterseite U, die bei der Untersuchung am Patienten, insbesondere seiner Zunge, anliegt. Die entgegengesetzte Seite des Grundkörpers 34 stellt die Oberseite O dar.

Der Grundkörper 34 hat im gezeigten Ausführungsbeispiel eine Macintosh-Form, d. h., dass die Unterseite U des Grundkörper 34 eine Krümmung hat. Der Grundkörper 34 kann selbst verständlich auch eine andere Form haben, wie eine Miller-Form, Dörges-Form oder McCoy-Form.

Die Einführrichtung R erstreckt sich von Griffende G zum Patientenende P hin. In horizontaler Querrichtung zur Einführrichtung R hat der Spatel 26 eine Breite B (Figur 3).

Im Grundkörper 34 ist ein Kanal 36 für den Kameraarm 20 ausgebildet. Der Kanal 36 erstreckt sich vom Griffende G auf der Oberseite O des Spatels 26 zum Patientenende P hin.

Zum Beispiel endet der Kanal 36 im mittleren Drittel zwischen dem Griffende G und dem Patientenende P. Der übrige Teil des Grundkörpers 34 zwischen dem Ende des Kanals 36 und dem Patientenende P ist somit ein Vorsprung 38 oder eine Zunge.

In Querrichtung erstreckt er sich über die halbe Breite B oder weniger des Grundkörpers 34, insbesondere asymmetrisch und/oder von einer Seite ausgehend.

Der Kanal 36 wird entlang seines Umfangs durch Kanalwände, die Teil des Grundkörpers 34 sind, begrenzt. Beispielsweise ist der Kanal 36 entlang seines Umfangs vollständig geschlossen. Im gezeigten Ausführungsbeispiel ist der Kanal 36 über seine gesamte Länge in Einführrichtung R entlang seines Umfangs vollständig geschlossen.

Der Kanal 36 weist an seinem dem Patientenende P zugewandten Ende eine erste Öffnung, griffseitige Öffnung 40 genannt, sowie zum Patientenende P hin eine zweite Öffnung auf, patientenseitige Öffnung 42 genannt. Die griffseitige Öffnung 40 und die patientenseitige Öffnung 42 sind insbesondere die einzigen Öffnungen des Kanals 36.

Im gezeigten Ausführungsbeispiel ist die patientenseitige Öffnung 42 kleiner als die griffseitige Öffnung 40. Der Querschnitt des Kanals 36 verkleinert sich somit von der griffseitigen Öffnung 40 zur patientenseitigen Öffnung 42 hin, insbesondere stetig.

Zur Änderung des Querschnitts kann die Ausdehnung des Kanals 36 in Querrichtung gleich bleiben und sich nur die Höhe des Querschnitts des Kanals 36, d. h. die Ausdehnung senkrecht zur Querrichtung und senkrecht zur Einführrichtung R, verkleinern.

Durch die patientenseitige Öffnung 42 ist der Kanal 36 insbesondere nicht verschlossen, wie es im Stand der Technik beispielsweise mittels einer Scheibe üblich ist.

Der Querschnitt des Kanals 36 ist aber so gewählt, dass der Kameraarm 20 in den Kanal 36 bis zur patientenseitigen Öffnung 42 eingeführt werden kann.

In den Figuren 5, 6 und 7 ist die Schutzkappe 28 in einer perspektivischen Darstellung, in einer Vorderansicht, d. h. auf das patientenseitige Ende, und in einer Schnittansicht gezeigt.

Die Schutzkappe 28 ist ein vom Spatel 26 separates Bauteil und kann ebenfalls einstückig und oder als Spritzgussteil ausgeführt sein. Das Material der Schutzkappe 28 ist transparent, insbesondere ein transparenter Kunststoff.

Die Schutzkappe 28 hat einen Schutzabschnitt 44 und einen Befestigungsabschnitt 46, wobei im gezeigten Ausführungsbeispiel der Befestigungsabschnitt 46 griffseitig an den Schutzabschnitt 44 anschließt.

Der Schutzabschnitt 44 weist eine transparente Frontscheibe 48 auf, die insbesondere eine zur patientenseitigen Öffnung 42 des Kanals 36 komplementäre Form hat.

Von der Frontscheibe 48 stehen zum Griffende hin vier Wände 50 ab, die insbesondere zueinander parallel sind.

Die Wände 50 und die Frontscheibe 48 schließen im gezeigten Ausführungsbeispiel jedoch keinen rechten Winkel ein, sodass die Frontscheibe 48 schräg zu den Wänden 50 angeordnet ist. Denkbar ist es auch, dass die Frontscheibe 48 senkrecht zu den Wänden 50 steht.

Die Wände 50 bilden zusammen mit der Frontscheibe 48 eine Aufnahme 52 für die Spitze des Kameraarms 20. Die Aufnahme 52 ist patientenseitig vollständig verschlossen, insbesondere von der Frontscheibe 48.

Griffseitig an die Wände 50 schließt der Befestigungsabschnitt 46 an.

Der Befestigungsabschnitt 46 weist wenigstens ein Befestigungsmittel 54 auf, mithilfe derer die Schutzkappe 28 im Kanal 36 positioniert werden kann.

Im gezeigten Ausführungsbeispiel sind die Befestigungsmittel 54 vier Flügel 56, wobei sich jeweils ein Flügel 56 von jeweils einer der Wände 50 zum Griffende G und zugleich nach außen erstreckt.

Alternativ oder zusätzlich können als Befestigungsmittel 54 abbrechbare Stege, eine dünne Kunststoffhaut, Klebstoff, insbesondere ein Klebstofftropfen, und/oder eine Folie eingesetzt werden.

Die Flügel 56 erstrecken sich insbesondere weiter nach außen in Bezug auf eine Mittellinie der Aufnahme 52 und oder weiter nach außen als der Schutzabschnitt 44.

Die Flügel 56 sind elastisch ausgeführt, sodass sie nach innen bewegt werden können.

In Figur 8 ist die Aufsatzbaugruppe 14 in einem Ausgangszustand dargestellt. In diesem Ausgangszustand ist die Schutzkappe 28 im Kanal 36 eingesetzt.

Die Schutzkappe 28 befindet sich in einer Ausgangsposition, die zwischen der griffseitigen Öffnung 40 und der patientenseitigen Öffnung 42 liegt. Insbesondere erstreckt sich in der Ausgangsposition kein Teil der Schutzkappe 28 durch die patientenseitige Öffnung 42.

Die Schutzkappe 28 ist durch die Befestigungsmittel 54, im gezeigten Ausführungsbeispiel also die Flügel 56, in der Ausgangsposition positioniert.

Im gezeigten Ausführungsbeispiel geschieht dies dadurch, dass die Flügel 56 an den Kanalwänden des Kanals 36 anliegen und gegen die Kanalwände aufgrund ihrer Elastizität vorgespannt sind. Somit wird die Schutzkappe 28 in ihrer Ausgangsposition verklemmt und gegen herausfallen gesichert.

Alternativ oder zusätzlich können abbrechbare Stege, eine dünne Kunststoffhaut, Klebstoff, insbesondere ein Klebstofftropfen, und/oder eine Folie die Schutzkappe 28 in der Ausgangsposition halten.

Die Positionierung in der Ausgangsposition ist in jedem Falle lösbar, d. h., dass die Schutzkappe 28 durch normale, bewusste Benutzung bewegt werden kann.

Beispielsweise werden die Schutzkappe 28 und der Spatel 26 separat hergestellt und die Schutzkappe 28 anschließend werksseitig im Kanal 36 des Spatels 26 in der Ausgangsposition positioniert. Der Ausgangszustand entspricht somit auch dem Verkaufszustand.

Zur Verwendung des Videolaryngoskopsystems 10 führt der Benutzer eine Aufsatzbaugruppe 14 im Ausgangszustand mit dem Videolaryngoskops 12 zusammen. Genauer gesagt führt er den Kameraarm 20 in den Kanal 36 des Spatels 26 ein.

Die Spitze des Kameraarms 20 trifft dann, wie in Figur 9 dargestellt, auf die Schutzkappe 28 in ihrer Ausgangsposition. Dabei greift die Spitze des Kameraarms 20 in die Aufnahme 52 ein, wobei der Kameraarm 20 durch die Flügel 56 in die Aufnahme 52 hinein geführt wird.

Der Benutzer führt anschließend den Kameraarm 20 noch weiter in den Kanal 36 ein, wodurch die Positionierung der Schutzkappe 28 gelöst und die Schutzkappe 28 aus ihrer Ausgangsposition relativ zum Grundkörper 34 in Richtung zur patientenseitigen Öffnung 42 des Kanals 36 bewegt wird.

In anderen Worten nimmt der Kameraarm 20 die Schutzkappe 28 aus ihrer Ausgangsposition zur patientenseitigen Öffnung 42 hin mit.

In Figur 10 ist der Benutzungszustand dargestellt, in dem der Kameraarm 20 vollständig, d. h. zur ordnungsgemäßen Verwendung notwendigen Länge in den Kanal 36 eingeführt wurde.

In diesem Benutzungszustand ist die Schutzkappe 28 im Bereich des patientenseitigen Endes des Kanals 36 angeordnet und erstreckt sich im gezeigten Ausführungsbeispiel zumindest teilweise durch die patientenseitige Öffnung 42 hindurch.

Der Kameraarm 20 hält sowohl den Spatel 26 als auch die Schutzkappe 28.

Gut zu erkennen ist, dass die Frontscheibe 48 durch die patientenseitige Öffnung 42 ragt. Zur Festlegung des Benutzungszustands können im Bereich des patientenseitigen Endes des Kanals 36 ein oder mehrere Anschläge 58 vorgesehen sein, die sich in den Kanal 36 erstrecken und an denen die Schutzkappe 28 anliegt, beispielsweise die Wände 50 der Schutzkappe 28 anliegen.

Im Benutzungszustand ist somit die Kamera 22 in der Aufnahme 52 der Schutzkappe 28 aufgenommen und wird durch diese vor Verunreinigungen geschützt. Der übrige Kameraarm 20 wird durch den Grundkörper 34 vor Verunreinigungen geschützt.

Das Blickfeld der Kamera 22 verläuft im Benutzungszustand durch die Frontscheibe 48 in Richtung des Patientenendes P.

Auf diese Weise wird der notwendige Schutz der Kamera 22 gewährleistet, ohne dass die Qualität der Aufnahme beeinträchtigt wäre. Gleichzeitig kann das Material des Spatels 26 unabhängig vom Material der Schutzkappe 28 gewählt werden, wodurch die Anforderungen an die Materialeigenschaften des Spatels reduziert werden. Dadurch können auch intransparente und recycelte Materialien für den Spatel 26 zum Einsatz kommen, wodurch zum Umweltschutz beigetragen wird.

Nach der Untersuchung wird der Kameraarm 20 aus dem Spatel 26, genauer gesagt dem Kanal 36, entfernt. Dabei kann die Schutzkappe 28 im Kanal 36 verbleiben oder zusammen mit dem Kameraarm 20 hinaus gezogen werden. Im ersteren Fall erhält man eine einfachere Benutzung des Spatels 26, im letzteren Fall ist es möglich, den Spatel 26 und die Schutzkappe 28 getrennt voneinander zu entsorgen, wodurch ein verbessertes Recycling möglich ist.

Anstelle der Positionierung der Schutzkappe 28 in einem Ausgangszustand im Kanal 36 ist es in einer alternativen Ausführungsform denkbar, dass der Kanal 36 zunächst leer ist und die Schutzkappen 28 in dem optionalen Spender 16 bereitgestellt werden.

Vor der Benutzung wird dann mittels des Spenders 16 eine Schutzkappe 28 auf der Spitze des Kameraarms 20 appliziert und der Kameraarm 20 mitsamt der Schutzkappe 28 in den Kanal 36 eingeführt bis der Benutzungszustand erreicht ist.

Dazu befinden sich im Gehäuse 30 des Spenders 16 eine Vielzahl an Schutzkappen 28, die derart hinter der Spendeöffnung 32 angeordnet sind, dass beispielsweise durch Einführen des Kameraarms 20 durch die Spendeöffnung 32 eine Schutzkappe 28 an der Spitze des Kameraarms 20 befestigt wird.

Alternativ kann wenigstens eine Schutzkappe 28 durch die Spendeöffnung 32 hinausragen, sodass der Kameraarm 20 in sie eingreifen kann.

## Patentansprüche

1. Aufsatzbaugruppe für einen Kameraarm (20) eines Videolaryngoskops (12) mit einem Spatel (26) und einer Schutzkappe (28),
wobei der Spatel (26) einen Grundkörper (34) mit einem Griffende (G) und einem zu dem Griffende (G) entgegengesetzten Patientenende (P) sowie einen im Grundkörper (34) ausgebildeten Kanal (36) zur Aufnahme des Kameraarms (20) aufweist, wobei der Kanal (36) sowohl zum Griffende (G) hin als auch zum Patientenende (P) hin geöffnet ist,
wobei die Schutzkappe (28) ein vom Spatel (26) separates Bauteil ist, in den Kanal (36) einführbar ist und einen zumindest teilweise transparenten Schutzabschnitt (44) aufweist, wobei die Schutzkappe (28) dazu ausgebildet ist, dass eine Spitze des Kameraarms (20) des Videolaryngoskops (12) in den Schutzabschnitt (44) eingreifen kann und vom Schutzabschnitt (44) abgedeckt wird,
**dadurch gekennzeichnet, dass** die Schutzkappe (28) im Ausgangszustand der Aufsatzbaugruppe (14) innerhalb des Kanals (36) angeordnet ist und einen Befestigungsabschnitt (46) aufweist, der die Schutzkappe (28) gegenüber dem Grundkörper (34) in einer Ausgangsposition positioniert.

2. Aufsatzbaugruppe nach Anspruch 1, **dadurch gekennzeichnet, dass** die Schutzkappe (28) im Benutzungszustand der Aufsatzbaugruppe (14) im Bereich des patientenseitigen Endes des Kanals (36) angeordnet ist, insbesondere sich zumindest teilweise durch die patientenseitige Öffnung (42) des Kanals (36) erstreckt.

3. Aufsatzbaugruppe nach Anspruch 1 oder 2, **dadurch gekennzeichnet, dass** der Befestigungsabschnitt (46) die Schutzkappe (28) gegenüber dem Grundkörper (34) in einer Ausgangsposition im Kanal (36) verklemmt.

4. Aufsatzbaugruppe nach einem der vorhergehenden Ansprüche, **dadurch gekennzeichnet, dass** der Befestigungsabschnitt (46) wenigstens zwei, insbesondere vier Flügel (56) aufweist, die sich von entgegengesetzten Seiten des Schutzabschnitts (44) aus erstrecken, wobei die wenigstens zwei Flügel (56) an entsprechenden Kanalwänden des Kanals (36) anliegen und/oder wobei sich die Flügel (56) weiter nach außen erstrecken als der Schutzabschnitt (44).

5. Aufsatzbaugruppe nach einem der vorhergehenden Ansprüche, **dadurch gekennzeichnet, dass** im Kanal (36) an seinem patientenseitigen Ende wenigstens ein Anschlag (58) vorgesehen ist, an dem die Schutzkappe (28), insbesondere der Schutzabschnitt (44), im Benutzungszustand anliegt.

6. Aufsatzbaugruppe nach einem der vorhergehenden Ansprüche, **dadurch gekennzeichnet, dass** der Schutzabschnitt (44) eine transparente Frontscheibe (48) und wenigstens zwei, insbesondere vier Wände (50) aufweist, wobei sich die Wände (50) von der Frontscheibe (48) aus erstrecken und eine Aufnahme (52) für die Spitze des Kameraarms (20) bilden, insbesondere wobei die Wände (50) parallel verlaufen und/oder die Frontscheibe (48) schräg zu den Wänden (50) angeordnet ist.

7. Aufsatzbaugruppe nach einem der vorhergehenden Ansprüche, **dadurch gekennzeichnet, dass** der Spatel (26) aus einem intransparenten Material und/oder aus einem zumindest überwiegend, insbesondere vollständig recycleten Material ist.

8. Aufsatzbaugruppe nach einem der vorhergehenden Ansprüche, **dadurch gekennzeichnet, dass** die patientenseitige Öffnung (42) des Kanals (36) zwischen dem Griffende (G) und dem Patientenende (P) des Grundkörpers (34) angeordnet ist, insbesondere im mittleren Drittel zwischen dem Griffende (G) und dem Patientenende (P).

9. Aufsatzbaugruppe nach einem der vorhergehenden Ansprüche, **dadurch gekennzeichnet, dass** der Kanal (36) entlang seines Umfangs geschlossen ist, insbesondere über die gesamte Länge des Kanals (36), und/oder dass sich der Querschnitt des Kanals (36) von der griffseitigen Öffnung (40) zur patientenseitigen Öffnung (42) des Kanals (36) hin verkleinert, insbesondere sich die Höhe des Querschnitts verkleinert.

10. Aufsatzbaugruppe nach einem der vorhergehenden Ansprüche, **dadurch gekennzeichnet, dass** der Grundkörper (34) in Querrichtung zur Einführrichtung (R) eine Breite (B) hat, wobei sich der Kanal (36) nur über einen Teil der Breite (B), insbesondere gleich oder weniger als die Hälfte der Breite (B) erstreckt.

11. Aufsatzbaugruppe nach einem der vorhergehenden Ansprüche, **dadurch gekennzeichnet, dass** der Grundkörper (34) einstückig ausführt ist und/oder dass die Schutzkappe (28) einstückig ausgeführt ist.

12. Videolaryngoskopsystem mit einem Videolaryngoskop (12), das einen Kameraarm (20) aufweist, und einer Aufsatzbaugruppe (14) für den Kameraarm nach einem der vorhergehenden Ansprüche.

13. Videolaryngoskopsystem nach Anspruch 12, **dadurch gekennzeichnet, dass** der Kameraarm (20) eine Spitze mit einer Kamera (22) aufweist, wobei im Benutzungszustand die Spitze in die Schutzkappe (28), insbesondere die Aufnahme (52) der Schutzkappe (28), eingreift und der Kameraarm (20) sich durch den Kanal (36) erstreckt.

14. Videolaryngoskopsystem nach Anspruch 12 oder 13, **dadurch gekennzeichnet, dass** das Videolaryngoskopsystem (10) derart ausgebildet ist, dass der Kameraarm (20) die Schutzkappe (28) beim Einführen des Kameraarms (20) in den Kanal (36) des Spatels (26) aus der Ausgangsposition mitnimmt und zur patientenseitigen Öffnung (42) des Kanals (36) schiebt, bis der Benutzungszustand erreicht ist.

15. Videolaryngoskopsystem nach Anspruch 13, **dadurch gekennzeichnet, dass** das Videolaryngoskopsystem (10) einen vom Kameraarm (20) und vom Spatel (26) separaten Spender (16) für die Schutzkappe (28) aufweist, der ein Gehäuse (30) mit einer Spendeöffnung (32) hat, wobei innerhalb des Gehäuses (30) im Bereich der Spendeöffnung (32) die Schutzkappe (28) vorgesehen ist.

## Claims

1. Attachment assembly for a camera arm (20) of a video laryngoscope (12) having a spatula (26) and a protective cap (28),
wherein the spatula (26) has a base body (34) having a handle end (G) and a patient end (P) opposite the handle end (G) as well as a channel (36) made in the base body (34) for receiving the camera arm (20), wherein the channel (36) is open both towards the handle end (G) as well as towards the patient end (P),
wherein the protective cap (28) is a component separate from the spatula (26), can be inserted into the channel (36) and has an at least partially transparent protective section (44), wherein the protective cap (28) is designed so that a tip of the camera arm (20) of the video laryngoscope (12) can engage into the protective section (44) and is covered by the protective section (44),
**characterised in that** the protective cap (28) is arranged inside the channel (36) in the initial state of the attachment assembly (14) and has a fastening section (46), which positions the protective cap (28) in relation to the base body (34) in an initial position.

2. Attachment assembly according to claim 1, **characterised in that** the protective cap (28) is arranged in the region of the patient-side end of the channel (36) in the use state of the attachment assembly (14), in particular it extends at least partially through the patient-side opening (42) of the channel (36).

3. Attachment assembly according to claim 1 or 2, **characterised in that** the fastening section (46) wedges the protective cap (28) into the channel (36) in relation to the base body (34) in an initial position.

4. Attachment assembly according to one of the preceding claims, **characterised in that** the fastening section (46) has at least two, in particular four wings (56), which extend out from opposing sides of the protective section (44), wherein the at least two wings (56) rest against corresponding channel walls of the channel (36) and/or wherein the wings (56) extend further outwards than the protective section (44).

5. Attachment assembly according to one of the preceding claims, **characterised in that** at least one abutment (58) against which the protective cap (28), in particular the protective section (44), rests in the use state is provided in the channel (36) on its patient-side end.

6. Attachment assembly according to one of the preceding claims, **characterised in that** the protective section (44) has a transparent front pane (48) and at least two, in particular four walls (50), wherein the walls (50) extend out from the front pane (48) and form a receptacle (52) for the tip of the camera arm (20), in particular wherein the walls (50) run parallel and/or the front pane (48) is arranged diagonal to the walls (50).

7. Attachment assembly according to one of the preceding claims, **characterised in that** the spatula (26) is made from a non-transparent material and/or from an at least predominantly, in particular completely recycled material.

8. Attachment assembly according to one of the preceding claims, **characterised in that** the patient-side opening (42) of the channel (36) is arranged between the handle end (G) and the patient end (P) of the base body (34), in particular in the centre third between the handle end (G) and the patient end (P).

9. Attachment assembly according to one of the preceding claims, **characterised in that** the channel (36) is closed along its circumference, in particular over the entire length of the channel (36), and/or **in that** the cross-section of the channel (36) reduces in size from the handle-side opening (40) towards the patient-side opening (42) of the channel (36), in particular the height of the cross-section reduces in size.

10. Attachment assembly according to one of the preceding claims, **characterised in that** the base body (34) has a width (B) in the transverse direction to the insertion direction (R), wherein the channel (36) only extends over a part of the width (B), in particular equal to or less than half of the width (B).

11. Attachment assembly according to one of the preceding claims, **characterised in that** the base body (34) is designed in one piece and/or **in that** the protective cap (28) is designed in one piece.

12. Video laryngoscope system having a video laryngoscope (12), which has a camera arm (20), and an attachment assembly (14) for the camera arm according to one of the preceding claims.

13. Video laryngoscope system according to claim 12, **characterised in that** the camera arm (20) has a tip with a camera (22), wherein in the use state the tip engages into the protective cap (28), in particular the receptacle (52) of the protective cap (28), and the camera arm (20) extends through the channel (36).

14. Video laryngoscope system according to claim 12 or 13, **characterised in that** the video laryngoscope system (10) is designed in such a way that, when the camera arm (20) is inserted into the channel (36) of the spatula (26), the camera arm (20) takes the protective cap (28) along with it from the initial position and slides it to the patient-side opening (42) of the channel (36) until the use state is reached.

15. Video laryngoscope system according to claim 13, **characterised in that** the video laryngoscope system (10) has a dispenser (16), separate from the camera arm (20) and from the spatula (26), for the protective cap (28), which dispenser has a housing (30) with a dispenser opening (32), wherein the protective cap (28) is provided in the region of the dispenser opening (32) inside the housing (30).

## Revendications

1. Module rapporté pour un bras de caméra (20) d'un laryngoscope vidéo (12) comprenant une lame (26) et un capuchon de protection (28),
dans lequel la lame (26) présente un corps de base (34) comportant une extrémité manche (G) et une extrémité patient (P) opposée à l'extrémité manche (G) ainsi qu'un canal (36) formé dans le corps de base (34) pour recevoir le bras de caméra (20), ledit canal (36) étant ouvert aussi bien vers l'extrémité manche (G) que vers l'extrémité patient (P),
dans lequel le capuchon de protection (28) est un composant distinct de la lame (26) qui peut être inséré dans le canal (36) et présente une partie de protection (44) au moins partiellement transparente, ledit capuchon de protection (28) étant conçu de manière qu'un bout du bras de caméra (20) du laryngoscope vidéo (12) puisse s'emboîter dans la partie de protection (44) et être recouverte par ladite partie de protection (44) ;
**caractérisé en ce que** le capuchon de protection (28) est disposé à l'intérieur du canal (36), à l'état initial du module rapporté (14), et présente une partie de fixation (46) qui positionne le capuchon de protection (28) dans une position initiale par rapport au corps de base (34).

2. Module rapporté selon la revendication 1, **caractérisé en ce que** le capuchon de protection (28), à l'état d'utilisation du module rapporté (14), est disposé à proximité de l'extrémité côté patient du canal (36) en s'étendant notamment au moins partiellement à travers l'ouverture côté patient (42) du canal (36).

3. Module rapporté selon la revendication 1 ou 2, **caractérisé en ce que** la partie de fixation (46) serre le capuchon de protection (28) par rapport au corps de base (34) dans une position initiale dans le canal (36).

4. Module rapporté selon l'une des revendications précédentes, **caractérisé en ce que** la partie de fixation (46) présente au moins deux, et notamment quatre, ailettes (56) s'étendant à partir de côtés opposés de la partie de protection (44), lesdites au moins deux ailettes (56) reposant contre des parois correspondantes du canal (36) et/ou lesdites ailettes (56) s'étendant davantage vers l'extérieur que la partie de protection (44).

5. Module rapporté selon l'une des revendications précédentes, **caractérisé en ce qu'**il est prévu dans le canal (36), au niveau de son extrémité côté patient, au moins une butée (58) contre laquelle repose le capuchon de protection (28), notamment la partie de protection (44), à l'état d'utilisation.

6. Module rapporté selon l'une des revendications précédentes, **caractérisé en ce que** la partie de protection (44) présente une fenêtre avant (48) transparente et au moins deux, et notamment quatre, parois (50), lesdites parois (50) s'étendant à partir de la fenêtre avant (48) et formant un réceptacle (52) pour le bout du bras de caméra (20), lesdites parois (50) étant notamment parallèles et/ou la fenêtre avant (48) étant disposée obliquement par rapport aux parois (50).

7. Module rapporté selon l'une des revendications précédentes, **caractérisé en ce que** la lame (26) se compose d'un matériau opaque et/ou d'un matériau qui est au moins majoritairement, et notamment totalement, recyclé.

8. Module rapporté selon l'une des revendications précédentes, **caractérisé en ce que** l'ouverture côté patient (42) du canal (36) est située entre l'extrémité manche (G) et l'extrémité patient (P) du corps de base (34), notamment dans le tiers médian entre l'extrémité manche (G) et l'extrémité patient (P).

9. Module rapporté selon l'une des revendications précédentes, **caractérisé en ce que** le canal (36) est fermé sur son pourtour, notamment sur toute la longueur du canal (36), et/ou **en ce que** la section transversale du canal (36) diminue depuis l'ouverture côté manche (40) jusqu'à l'ouverture côté patient (42) du canal (36), et notamment la hauteur de la section transversale diminue.

10. Module rapporté selon l'une des revendications précédentes, **caractérisé en ce que** le corps de base (34) présente une largeur (B) dans la direction transversale à la direction d'insertion (R), ledit canal (36) ne s'étendant que sur une partie de la largeur (B) qui est notamment inférieure ou égale à la moitié de la largeur (B).

11. Module rapporté selon l'une des revendications précédentes, **caractérisé en ce que** le corps de base (34) est réalisé d'une seule pièce et/ou **en ce que** le capuchon de protection (28) est réalisé d'une seule pièce.

12. Système de laryngoscope vidéo comprenant un laryngoscope vidéo (12), présentant un bras de caméra (20), et un module rapporté (14) pour bras de caméra selon l'une des revendications précédentes.

13. Système de laryngoscope vidéo selon la revendication 12, **caractérisé en ce que** le bras de caméra (20) a un bout doté d'une caméra (22), ledit bout s'emboîtant, à l'état d'utilisation, dans le capuchon de protection (28), notamment dans le réceptacle (52) du capuchon de protection (28), et ledit bras de caméra (20) s'étendant à travers le canal (36).

14. Système de laryngoscope vidéo selon la revendication 12 ou 13, **caractérisé en ce que** le système de laryngoscope vidéo (10) est conçu de telle façon que le bras de caméra (20) entraîne le capuchon de protection (28) hors de sa position initiale lorsque le bras de caméra (20) est inséré dans le canal (36) de la lame (26) et le pousse vers l'ouverture côté patient (42) du canal (36) jusqu'à ce que l'état d'utilisation soit atteint.

15. Système de laryngoscope vidéo selon la revendication 13, **caractérisé en ce que** le système de laryngoscope vidéo (10) présente un distributeur (16) de capuchon de protection (28) distinct du bras de caméra (20) et de la lame (26) et qui comporte un boîtier (30) pourvu d'une ouverture de distribution (32), ledit capuchon de protection (28) étant prévu à l'intérieur du boîtier (30) à proximité de l'ouverture de distribution (32).
